Europäisches Patentamt

European Patent Office

Office européen des brevets

(11) Publication number: **0 428 141 A2**

# EUROPEAN PATENT APPLICATION

(21) Application number: 90121728.1

(22) Date of filing: 13.11.90

(51) Int. Cl.5: **C12N 15/81**, C12N 15/67, C12P 21/02

(30) Priority: **14.11.89 JP 295818/89**

(43) Date of publication of application:
**22.05.91 Bulletin 91/21**

(84) Designated Contracting States:
**BE CH DE DK ES FR GB IT LI NL SE**

(71) Applicant: **THE GREEN CROSS CORPORATION**
**3-3, Imabashi 1-chome Chuo-ku**
**Osaka-shi Osaka 541(JP)**

(72) Inventor: **Ishida, Yutaka, c/o The Green Cross**
**Corporation**
**Central Research Lab., 2-1180-1,**
**Shodaiohtani**
**Hirakata-shi, Osaka 573(JP)**
Inventor: **Murakami, Koji, c/o The Green Cross**
**Corporation**
**Central Research Lab., 2-1180-1,**
**Shodaiohtani**
**Hirakata-shi, Osaka 573(JP)**
Inventor: **Hayasuke, Naofumi, c/o The Green**
**Cross Corporation**
**Central Research Lab., 2-1180-1,**
**Shodaiohtani**
**Hirakata-shi, Osaka 573(JP)**
Inventor: **Nakagawa, Yukimitsu, c/o The Green**
**Cross Corp.**
**Central Research Lab., 2-1180-1,**
**Shodaiohtani**
**Hirakata-shi, Osaka 573(JP)**
Inventor: **Kawabe, Haruhide, c/o The Green**
**Cross Corp.**
**Central Research Lab., 2-1180-1,**
**Shodaiohtani**
**Hirakata-shi, Osaka 573(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

(54) Hybrid promoter.

(57) An improved yeast promoter wherein HSA productivity is greatly increased by combining SUC2-UAS and GAP-DH promoter, and production method thereof are discussed.

## HYBRID PROMOTER

### FIELD OF THE INVENTION

The present invention relates to an improved yeast promoter.

### BACKGROUND OF THE INVENTION

It is a known fact that the control mechanism of yeast promoter is generally based on positive control with the exception of glucose suppression and so on. It is suggested that a cis-functioning upstream activation site (hereinafter referred to as UAS), as a promoter side control factor involved in this positive control, is present several hundred bases upstream the translation initiation point [L. Guarente: Cell, *36*, 799 (1984)] .

Also, it is shown that substitution of this UAS with another UAS frees the promoter from the starting control system and makes it enter under the control related to the substitution product UAS [L. Guarente et al.: Proc. Natl. Acad. Sci. USA, *79*, 7410 (1982); L. Guarente et al.: Cell, *36*, 503 (1984); K. Struhl: Proc. Natl. Acad. Sci. USA, *81*, 7865 (1985) ] . Therefore, in an attempt to improve a yeast promoter, the control system may be modified by UAS substitution, or the promoter may be freed from the control system by DNA deletion or the promoter size may be miniaturized. Examples of yeast promoters include GAP-DH (glyceraldehyde-3-phosphate dehydrogenase gene) promoter which is utilized as a plasmid for the production of hepatitis B virus surface antigen (HBsAg) [e.g. expression of heterologous genes in *Saccharomyces cerevisiae* from vector utilizing the GAP-DH promoter described by Bitler, G. A. and Egan, K. M. in Gene, *32*, 263-274 (1984); the yeast expression vector and method of its use described in Japanese Patent Publication Open to Public Inspection No. 210888/1984] .

For example, HBsAg-producing plasmid pGG5 was constructed with GAP-DH promoter, HBsAg structural gene, GAP-DH terminator, origin of replication in *Escherichia coli*, marker gene (ampicillin resistance gene (Apc) in *Escherichia coli* and leucine gene in yeast), and origin of replication in yeast.

Also, examples of yeast promoters include SUC2 (yeast invertase gene) promoter (Japanese Patent Publication Open to Public Inspection Nos. 41488/1985 and 224291/1987) which is utilized to produce interferon, etc.

### SUMMARY OF THE INVENTION

With the aim of producing human serum albumin (hereinafter referred to as HSA) using a yeast host system, the present inventors investigated various systems for its expression. Various promoters including GAP-DH promoter, SUC2 promoter, etc. were examined, but none of them gave a satisfactory production.

### BRIEF DESCRIPTION OF THE DRAWINGS

Fig. 1 shows the procedure of making the yeast SUC2 gene promoter UAS domain a cassette.
Fig. 2 shows the base sequence of the SUC2-UAS domain.
Fig. 3 shows the procedure of construction of HSA yeast extracellular secretory expression vector pYI032 using a hybrid promoter comprising the SUC2-UAS domain and the GAP-DH TATA box.
Fig. 4 shows the restriction enzyme map of HSA yeast extracellular secretory expression vector pYI032.

### DETAILED DESCRIPTION OF THE INVENTION

The present inventors prepared a hybrid promoter by ligating a domain containing the UAS of SUC2 promoter and another domain containing the TATA box of GAP-DH promoter, and attempted its expression.

The present inventors thus found that strong promoter activity was expressed by said promoter, and developed the present invention.

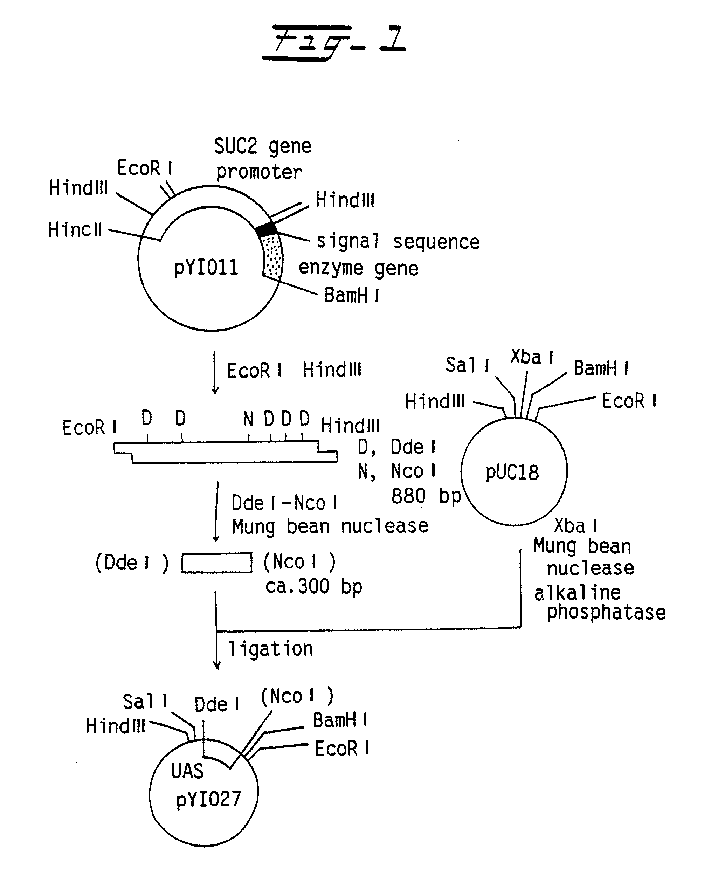

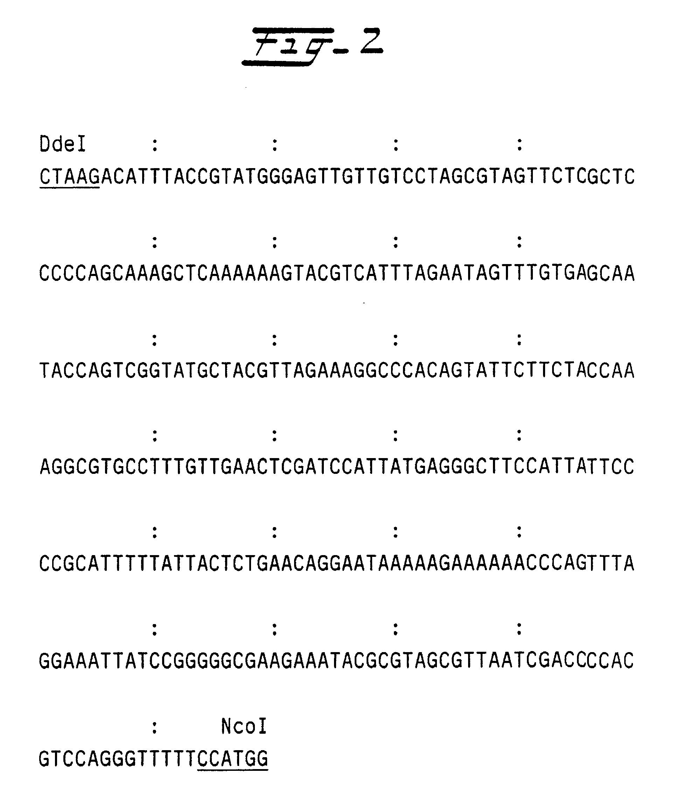

The SUC2 promoter used for the present invention can be obtained from a yeast chromosomal DNA on the basis of the already-reported base sequence of SUC2 gene (Japanese Patent Publication Open to Public Inspection No. 41488/1985). Base sequence analysis reveals that digestion of the yeast chromosomal DNA with HincII-BamHI will yield a 2.56 kb fragment comprising the promoter domain of SUC2 gene, a signal sequence and a part of enzyme gene. Then, a yeast chromosomal DNA library was obtained by inserting a 2.3 to 3.2 kb fragment selected out of the HincII-BamHI digestion products of the yeast chromosomal DNA into pUC18 at the HincII-BamHI site. From this library, a clone considered to have the SUC2 gene was identified by colony hybridization using as a probe a 50 mer oligonucleotide 5'-ACT AGC GAT AGA CCT TTG GTC CAC TTC ACA CCC AAC AAG GGC TGG ATG AA-3' synthesized on the basis of a partial sequence of the invertase-encoding domain, whereby a plasmid named pYI011 was obtained (Fig. 1). The UAS of SUC2 promoter is known to be located in the range of from the -418 to -650 positions upsteam the translation initiation point [Sarokin, L and Carlson, M: Mol. Cell. Biol., 5, 2521 (1985)]. The DNA base sequence of the SUC2-UAS domain is shown in Fig. 2. Taking note of this fact, a domain containing these was digested with an appropriate restriction enzyme (e.g. -695 DdeI site and -386 NcoI site) to make the SUC2-UAS domain a cassette. Specifically, pYI011 was digested with DdeI-NcoI and then subjected to mung bean nuclease digestion [Kowalski, D., Kroeker, W. D. and Laskowski, M. Sr.: Biochemistry, 15, 4457 (1976)], and an about 300 bp blunt-ended fragment was separated. This fragment was inserted into pUC18, whose ends had been blunted with mung bean nuclease, at the XbaI site to yield pYI027, a plasmid wherein the DdeI site side is ligated to the SalI site side of the polylinker portion (Fig. 1).

GAP-DH promoter is located at the -674 to -24 position in the GAP-DH gene, and a miniaturized promoter thereof (-164 to -25) is also in public knowledge (Japanese Patent Publication Open to Public Inspection No. 175180/1987). The TATA box of GAP-DH promoter is located at the -140 position. The plasmid carrying this miniaturized promoter was designated as pGG7.

These two plasmids are subjected to ligation (e.g. BglII-BamHI treatment) to obtain a 150 bp fragment containing the TATA box of GAP-DH promoter, which is then inserted into a plasmid carrying SUC2-UAS (e.g. pYI027) at the BamHI site to yield a hybrid promoter (pYI030 in Example). This plasmid can be used as a cassette for expression as described below. As desired, it can be ligated with a plasmid carrying a structural gene, a signal sequence and a terminator sequence to yield a vector for secretory expression of a particular protein. In Example, HSA was secretorily expressed.

HSA sectretory expression vector pYI032, harboring the SUC2-UAS/mGAP-DH promoter • mHSA signal sequence was introduced into yeast strain AH22 and examined for its HSA productivity. As a result, this strain secreted 120 μg/mℓ HSA in the medium as determined by the RPHA method, which represents a productivity 3 times that obtained using vector pYI031 not containing the SUC2-UAS domain, and 4 times that obtained using vector pNH016 containing the entire SUC2 promoter. These findings demonstrate that the combination of SUC2-UAS and mGAP-DH promoters is quite effective in the production of HSA.

## EXAMPLE

(1) Construction of HSA yeast extracellular secretory expression vector, using SUC2-UAS/mGAP-DH promoter

i) Materials and method

(a) Plasmids

pYI027: A plasmid obtained by inserting a DdeI-NcoI fragment containing SUC2-.AS after blunting the ends with mung bean nuclease into pUC18, whose ends had been blunted in the same manner at the XbaI site (Fig. 1).

pGG7: HBsAg yeast intracellular expression vector carrying a miniaturized GAP-DH (mGAP-DH) promoter (Japanese Patent Publication Open to Public Inspection No. 175180/1987).

pUC18: Messing, J.: Methods in Enzymology, 101, 20 (1983).

pYN026: HSA yeast extracellular secretory expression vector harboring the GAL1 promoter modified HSA signal sequence (mHSA signal sequence) (EP Publication No. 319641).

(b) Strain

E. coli JM109 competent cells produced by Takara Shuzo were used.

(c) Recombinant DNA techniques

E. coli JM109 was transformed in accordance with the protocol of Takara Shuzo. DNA fragment ligation was conducted using a DNA ligation kit produced by Takara Shuzo. Other recombinant DNA techniques such as plasmid preparation, restriction enzyme treatment and agarose gel electrophoresis were carried out in accordance with standard methods.

ii) Results

(a) Preparation of HSA secretory expression vector pYI032 using SUC2-UAS/mGAP-DH promoter

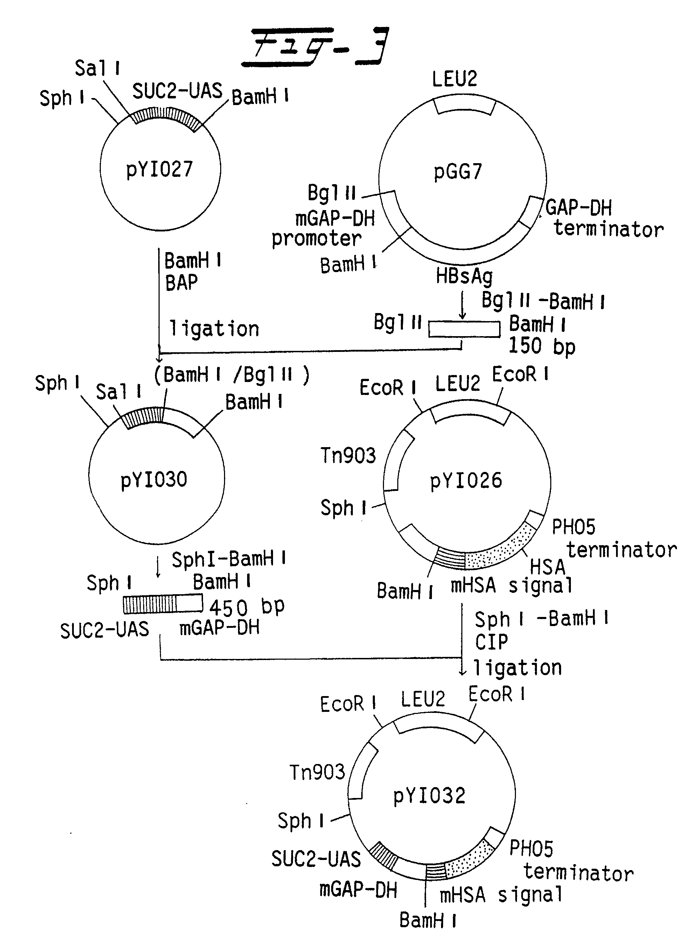

The schematic of HSA secretory vector pYI032 using SUC2- UAS/mGAP-DH promoter is shown in Fig. 3.

First, pGG7 was digested with BglII-BamHI to yield an about 150 bp DNA fragment containing the mGAP-DH promoter, which was then inserted into pYI027 at the BamHI site. Plasmids were prepared from 12 of a number of transformants thus obtained, and digested with SalI-BamHI. If the about 150 bp DNA fragment containing the mGAP-DH promoter has been inserted in the desired direction (if the BamIII site side of the fragment containing the mGAP-DH promoter has been ligated to the SUC2-UAS side), an about 450 bp DNA fragment which comprises SUC2-UAS and the mGAP-DH promoter occurs by SalI-BamHI digestion. Of the 12 strains examined, 4 were found to have an about 450 bp DNA fragment, suggesting possession of pYI030.

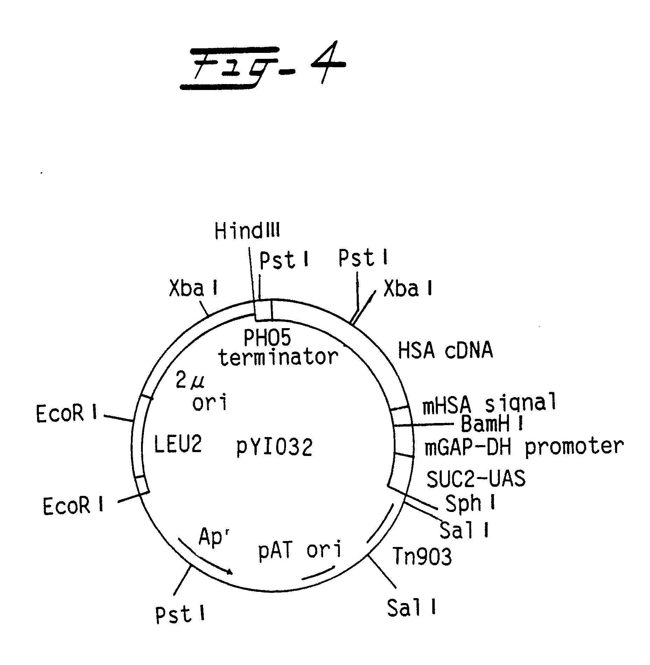

Then, pYI030 was digested with SphI-BamHI to yield an about 450 bp DNA fragment containing SUC2-UAS/mGAP-DH promoter. This fragment was then inserted into pYN026 at the SphI-BamHI site. Plasmids were prepared from 12 of a number of transformants thus obtained, and digested with EcoRI-BamHI. If the desired plasmid pYI030 has been obtained, it is expected that two fragments will occur, namely an about 800 bp fragment originating from a vicinity of the LEU2 gene produced by EcoRI-BamHI digestion and then an about 5 kb fragment produced by digestion at the BamHI site located between the promoter and the signal sequence. The plasmids prepared from these 12 transformants were digested with EcoRI. All of them showed the same digestion pattern as expected with pYI032, suggesting possession of pYI032. The pYI032 restriction enzyme map is shown in Fig. 4.

(2) Introduction of HSA yeast extracellular secretory expression vector into yeast and HSA production

i) Materials and method

(a) Strain

Saccharomyces cerevisiae AH22; a, his4, leu2, can1

(b) Plasmids

pYI031: An HSA secretory expression vector harboring mGAP-DH promoter * mHSA signal sequence which can be obtained by the same construction procedure as in Fig. 3 except that pUC18 is used in place of pYI027.

pYI032: An HSA secretory expression vector harboring SUC2-UAS/mGAP-DH promoter • mHSA signal sequence (Fig. 4).

pYI016: An HSA secretory expression vector harboring SUC2 promoter • mHSA signal sequence (positive control).

(c) Culture medium

YPF broth [2% Bacto peptone (Difco), 1% yeast extract (Difco), 2% fructose] and YNB broth [0.67% yeast nitrogen base (Defco), 2% glucose] were used as they were or with the addition of agar. The transformant regeneration medium used was prepared by adding 2% sorbitol to YNB agar medium.

(d) Yeast transformation

Transformation was carried out by the protoplast method [Hinnen, A., Hicks, J. B. and Fink, G. R.: Proc. Natl. Acad. Sci. USA, *75*, 1929 (1978)]. LEU2 was used as selection marker.

ii) Results

(a) Plasmid introduction into yeast

Using pNH016, pYI031 and pYI032, *S. cerevisiae* AH22 strain was transformed by the protoplast method. LEU2 was used as selection marker. As a result, irrespective of which plasmid was used, transformants were obtained with a yield of more than $10^3$ cells per µg plasmid.

(b) HSA secretion by transformants

The transformants obtained in (a) were examined for HSA secretory production. First, for 6 transformants for each plasmid, cells were collected from the colony that grew on the regeneration plate, and inoculated in 3 *mℓ* of YPF broth contained in a middle-sized test tube with an aluminum cap. After shaking culture at 30°C for 48 hours, the optical absorbance at 540 nm of the culture broth and the HSA concentration of the culture supernatant were determined. HSA determination was achieved by the RPHA method. None of the resulting clones showed extremely bad proliferation, and there was almost no dispersion in HSA production among the clones.

Then, flask culture was conducted to obtain an exact figure of HSA production. First, these strains were inoculated into 5 *mℓ* of YNB broth contained in a middle-sized test tube with an aluminum cap, followed by shaking culture at 30°C for 3 days. Then, the entire amount of this preculture broth was inoculated into 50 *mℓ* of YPF broth contained in a 300-*mℓ* conical flask with a baffle, followed by shaking culture at 30°C. The optical absorbance at 540 nm of the culture broth and HSA production were determined at given time intervals. HSA determination was achieved by the RPHA method (Table 1).

Table 1

| Strain (plasmid/host) | Promoter | HSA production (mg/ℓ) after 72 hours of culture in YPF broth |
|---|---|---|
| pYI032/AH22 | SUC2-UAS/mGAP-DH | 120 |
| pYI031/AH22 | mGAP-DH | 40 |
| pNH016/AH22 | SUC2 | 30 |

The HSA production was 120 mg/ℓ at 72 hours following initiation of pYI032/AH22 cultivation. It is therefore evident that the HSA production obtained when using mGAP-DH promoter (pYI031/AH22), namely 40 mg/ℓ, increased to 120 mg/ℓ by ligating the SUC2-UAS domain upstream the mGAP-DH promoter (pYI032/AH22). This productivity was fairly higher than that obtained using the entire domain of SUC2 promoter (pNH016/AH22). Based on these findings, it was confirmed that the combination of SUC2-UAS and mGAP-DH promoters serves as a quite excellent promoter in the production of HSA.

**Claims**

1. A hybrid promoter resulting from the ligation of a domain containing the UAS of SUC2 promoter and another domain containing the TATA box site of GAP-DH promoter.

2. A hybrid promoter as claimed in Claim 1 wherein said domain containing the UAS of SUC 2 promoter is from -695 DdeI site to -386 NcoI site of the SUC2 gene.

3. A hybrid promoter as claimed in Claim 1 wherein said domain containing the TATA box site of GAP-DH promoter is an about 150 bp fragment of plasmid carrying a miniaturized GAP-DH promoter.

4. A method of producing a heterologous protein using a hybrid promoter as claimed in Claim 1.

5. A method of producing a heterologous protein as claimed in Claim 4 which comprises insertion of a gene encoding the heterologous protein under the control of the hybrid promoter as claimed in Claim 1, thereby obtaining recombinant plasmid and transformation of a yeast, followed by expression.

6. A method of producing a heterologous protein as claimed in Claim 5 wherein the heterologous protein is human serum albumin (HSA).

Fig-1

# _Fig_- 2

```
DdeI       :           :           :           :
CTAAGACATTTACCGTATGGGAGTTGTTGTCCTAGCGTAGTTCTCGCTC


           :           :           :           :
CCCCAGCAAAGCTCAAAAAAGTACGTCATTTAGAATAGTTTGTGAGCAA


           :           :           :           :
TACCAGTCGGTATGCTACGTTAGAAAGGCCCACAGTATTCTTCTACCAA


           :           :           :           :
AGGCGTGCCTTTGTTGAACTCGATCCATTATGAGGGCTTCCATTATTCC


           :           :           :           :
CCGCATTTTTATTACTCTGAACAGGAATAAAAAGAAAAAACCCAGTTTA


           :           :           :           :
GGAAATTATCCGGGGGCGAAGAAATACGCGTAGCGTTAATCGACCCCAC


           :       NcoI
GTCCAGGGTTTTTCCATGG
```

Fig-3

Fig-4